# EUROPEAN PATENT SPECIFICATION

(11) **EP 3 143 963 B1**
(45) Date of publication and mention of the grant of the patent: **28.11.2018**
(21) Application number: 16183586.3
(22) Date of filing: 12.12.2013
(51) Int. Cl.: A61F 2/30, A61F 2/38, A61F 2/46

(54) **METHOD AND DEVICE FOR PRODUCING TEMPORARY KNEE JOINT SPACERS WITH STEMS OF VARIOUS SIZES**
VERFAHREN UND VORRICHTUNG ZUR HERSTELLUNG PROVISORISCHER KNIEGELENK-ABSTANDSHALTER MIT SCHÄFTEN UNTERSCHIEDLICHER GRÖSSE
PROCÉDÉ ET DISPOSITIF DE PRODUCTION D'ESPACEURS D'ARTICULATION DU GENOU TEMPORAIRE AVEC TIGES DE TAILLES DIVERSES

(30) Priority: 12.12.2012 US 201261736386 P
(43) Date of publication of application: 22.03.2017
(62) Divisional of application: 13818876.8
(73) Proprietor: Biomet Manufacturing, LLC, Warsaw, IN 46582 (US)
(72) Inventor: SMITH, Daniel B., Warsaw, IN 46580 (US); KREIDER, Tayler E., Warsaw, IN 46582 (US)
(74) Representative: Manitz Finsterwald Patentanwälte PartmbB

(56) References cited:
- WO-A1-2012/158618
- DE-A1-102011 104 808
- US-A1- 2004 036 189
- US-A1- 2010 102 484

## Description

### FIELD

This disclosure relates generally to temporary orthopedic implants for use in orthopedic surgical procedures and, more particularly, to cement molds for use in forming temporary orthopedic implants used in orthopedic surgical procedures.

### BACKGROUND

This section provides background information related to the present disclosure which is not necessarily prior art.

A natural joint may undergo degenerative changes due to a variety of etiologies. When these degenerative changes become so far advanced and irreversible, it may ultimately become necessary to replace the natural joint with a joint prosthesis. However, due to any number of reasons, a small portion of patients that undergo such orthopedic surgical procedures suffer from infections at the surgical site and generally around the implanted joint prosthesis. In order to eradicate or clear such an infection in a two-stage reimplantation, the implanted joint prosthesis is generally removed, the site is thoroughly debrided and washed, antibiotics are applied to the infected site until the infection is eliminated, and a new revision type joint prosthesis is then implanted during a subsequent orthopedic surgical procedure. Systemic antibiotics may also act as an adjunct to local antibiotic delivery. Another technique, more popular in Europe, is the one stage reimplantation in which the prosthesis is removed, the site is debrided and washed and a new permanent implant is cemented in place using antibiotic loaded bone cement. Prior art mold assemblies are found in the document US 2010/102484 A1.

The currently available techniques for delivering the antibiotic to the infected joint area include mixing appropriate bone cement, such as (PMMA) polymethyl-methacrylate, with an antibiotic, such as gentamicin, and applying the mixture to the infected joint area. Another technique involves the use of pre-loaded antibiotic cement beads which are retained on a string or wire. The antibiotic loaded bone cement is packed into the voids created by the explanted joint prosthesis while the joint is distracted or the string of antibiotic loaded beads are dropped into the respective voids. During this period, the antibiotic leaches out from the bone cement and into the infected area, while the patient may be left substantially non-ambulatory or bed-ridden with very limited mobility. In addition, soft tissue contraction in the area about the joint may cause a more difficult revision surgery since the remaining bone portion is smaller than the explanted joint prosthesis. Moreover, the above techniques may also suffer from the disadvantage of sometimes being difficult or messy to use during the orthopedic surgical procedure. This disadvantage is primarily exhibited during the use of the antibiotic loaded bone cement in a doughy state and attempting to fill the appropriate region in the distracted joint area.

### SUMMARY

This section provides a general summary of the disclosure, and is not a comprehensive disclosure of its full scope or all of its features.

The above disadvantages are overcome by a combination of three separate cement mold assemblies having the features as defined in claim 1. Such a combination of three separate cement mold assemblies, each configured to form a component of a temporary knee implant for use in delivering antibiotics to an infected site of a knee, comprises:
a tibial knee component mold assembly, the tibial knee component mold assembly having a second mold member receivable within a first mold member to define a tibial component mold cavity therebetween, the tibial component mold cavity defining a tibial tray surface and a tibial bearing surface and optionally a stem portion, and the second mold member being configured to be selectively and progressively indexed relative to the first mold member via cooperating engagement members until a desired thickness of the tibial component mold cavity is obtained;
a femoral knee component mold assembly, the femoral knee component mold assembly having a femoral component mold cavity formed between an inner sidewall defining a shape of a bone opposing surface and an outer side wall defining a shape of an articulating surface which contacts a tibial tray of a tibial component; and
an intramedullary canal filling component mold assembly, the intramedullary canal filling component mold assembly having an elongated intramedullary canal filling component mold cavity defined by a flexible elongated member enabling adjustment of the mold cavity to correspond to an intramedullary canal of an individual patient.

A cement mold assembly configured to form a temporary implant for use in delivering antibiotics to an infected site can include a first mold and a second mold. The first mold can have an open end and an inner wall. The first mold can define a tibial component forming cavity including a platform forming cavity and a stem forming cavity. The second mold can have a body portion configured to be slidably and progressively receivable by the inner wall into the tibial component forming cavity in a direction toward the closed end. Progressive advancement of the second mold into the tibial component forming cavity urges cement within the tibial component forming cavity against the body portion and the inner wall to form a unitary tibial component having a tibial tray portion formed by the platform forming cavity and a stem portion formed by the stem forming cavity.

According to additional features, the first mold can define a port opposite the open end that is configured to receive cement therethrough. The cement mold assembly can further comprise a first plate and a second plate positioned outboard of the first and second molds, respectively. The cement mold assembly can further comprise at least two rods connected between the first and second plates. The rods and the first and second plates can be selectively and progressively indexable. The first and second plates can be movable in discrete increments toward each other while the second mold is progressively advanced into the tibial component forming cavity. The rods can include teeth formed thereon that cooperatively engage an opposing surface on one of the first and second plates. The body of the second mold can include a geometry that substantially corresponds to a tibial bearing surface. The first mold can be formed of silicone. The second mold can be formed of silicone.

A cement mold assembly configured to form a temporary implant for use in delivering antibiotics to an infected site can include a first mold and a second mold. The first mold can have a first closed end, a second open end, and a sidewall. The first mold can define a cavity. The second mold can have a base portion and a cannulated member extending therefrom. The base portion can have a footprint complementary to the sidewall of the first mold such that the second mold is slidably and progressively receivable by the sidewall into the cavity in a direction toward the closed end. Progressive advancement of the second mold into the cavity can urge the cement within the cavity into the cannulated member. The first mold can cooperate with the second mold to form a unitary tibial component having a tibial tray portion formed by the cavity and a stem portion formed by the cannulated member.

According to additional features, the cement mold assembly can further comprise a reinforcement member configured to be placed at least partially within the cavity prior to progressive advancement of the second mold into the cavity. The reinforcement member can be configured to be molded into the unitary tibial component. The reinforcement member can comprise a first lateral portion that generally opposes a first closed end and a second longitudinal portion that is received by the cannulated member. The cannulated member can have a generally rectangular cross-section. The base portion of the second mold can have a textured surface. The first mold can include gradations on the sidewall. The first mold can be formed of silicone. The second mold can be formed of polyethylene. The reinforcement member can be formed of biocompatible metal.

According to still other features, a cement mold assembly configured to form a temporary implant for use in delivering antibiotics to an infected site can comprise a mold body having an outer sidewall and an inner sidewall that cooperate to define a cavity. The inner sidewall can be operable to define a shape of the temporary implant. The mold body can include articulating forming portions and a stem forming portion. The articulating and stem forming portions can cooperate to form a unitary femoral component having an articulating portion formed by the articulating forming portion and a stem portion formed by the stem forming portion.

According to other features, the stem forming portion can be removably coupled to the articulating forming portion. The stem forming portion can be threadably coupled to the articulating forming portion. The stem forming portion can have a first end defining first threads and a second end defining second threads. The first threads can be configured to threadably couple with the articulating forming portion. The second threads can be configured to threadably couple with a cement delivery device. The mold body can define vents therethrough. According to additional features, a reinforcement member can be configured to be placed at least partially within the cavity. The reinforcement member can be configured to be molded into the unitary femoral component.

A method of making a temporary knee implant according to the invention is defined in claim 9. A method of making a temporary implant for use in delivering antibiotics to an infected site can include, providing a first mold that defines an implant forming cavity collectively defined by an implant body forming cavity and an implant stem forming cavity. A second mold can be provided. Flowable material can be inserted into the implant forming cavity. At least one of the first and second molds can be advanced toward the other of the first and second molds causing the flowable material to be urged against the first mold. Subsequent to curing of the flowable material, the temporary implant can be removed from the first mold. The temporary implant can include an integrally formed implant body and implant stem formed by the respective implant body forming cavity and the implant stem forming cavity.

The implant forming cavity can comprise a tibial component forming cavity. The implant forming cavity according to other features can include a femoral component forming cavity. According to additional features, the method can include threadably coupling a flowable material delivery device to a connection port provided on a first plate. Advancing can comprise progressively engaging teeth extending from rods with a second plate.

A combination of three separate cement mold assemblies is provided, each being configured to form a component of a temporary knee implant for use in delivering antibiotics to an infected site of a knee. A tibial knee component mold assembly is characterized by a second mold member receivable within a first mold member to define a tibial component mold cavity therebetween. The tibial component mold cavity define a tibial tray surface and a tibial bearing surface and optionally a stem portion. The second mold member is configured to be selectively and progressively indexed relative to the first mold member via cooperating engagement members until a desired one of a plurality of thicknesses of the tibial component mold cavity is obtained. A femoral knee component mold assembly is characterized by a femoral component mold cavity formed between an inner sidewall defining a shape of a bone opposing surface and an outer side wall defining a shape of an articulating surface which contacts a tibial tray of a tibial component. An intramedullary canal filling component mold assembly is characterized by an elongated intramedullary canal filling component mold cavity defined by a flexible elongated member enabling adjustment of the mold cavity to correspond to an intramedullary canal of an individual patient.

According to other features, the tibial bone opposing surface of the tibial component mold assembly can comprise a textured surface. One of the first mold member and the second mold member of the tibial component mold assembly can comprise gradations. The inner sidewall can comprise an inner layer comprising silicone coupled to an outer layer comprising a material that is more rigid than silicone, and optionally the rigid material is one of a polycarbonate, a polyamide, and a copolyester. A threaded coupling can provide a fill port into the femoral component mold cavity at a position that is lateral of a portion of the articulating surface which primarily contacts a tibial tray of a tibial component, and is optionally lateral of the portion of the articulating surface which primarily contacts the tibial tray of the tibial component.

According to other features, the elongated intramedullary canal filling component mold cavity of the intramedullary canal filling component mold assembly can comprise an elongated, cylindrical mold cavity and a reduced-dimension, axially extending cavity at one end. The intramedullary canal filling component mold assembly can comprise a radiused surface at an insertion end opposite the one trailing end to facilitate insertion of a molded stem component in an elongated bone cavity. The flexible elongated member of the intramedullary canal filling component mold assembly can be at least semi-transparent to permit an x-ray to be viewed through the flexible elongated member.

A method of making a temporary knee implant is characterized by obtaining a tibial knee component mold assembly characterized by a first mold member and a second mold member. The second mold member is inserted into the first mold member to define a tibial knee component mold cavity including a tibial platform forming portion, and optionally a stem forming portion. At least one of the first and second molds is advanced toward the other of the first and second molds by successively engaging cooperating engagement members of the first and second mold members until a desired thickness of the platform forming portion is obtained. A femoral knee component mold assembly is obtained that is characterized by a femoral component mold cavity formed between an inner sidewall defining a shape of a bone opposing surface and an outer side wall defining a shape of an articulating surface for contacting a tibial tray of a tibial knee component. A separate intramedullary canal filling component mold assembly is obtained that is characterized by an elongated intramedullary canal filling component mold cavity defined by a flexible elongated member enabling adjustment of the mold cavity to correspond to an intramedullary canal of an individual patient.

According to other features, flowable antibiotic laden bone cement can be inserted into each of the tibial knee component mold cavity, the femoral component mold cavity, and the intramedullary canal filling component mold cavity through a port in each of the respective tibial knee component mold assembly, femoral component mold assembly, and intramedullary canal filling component mold assembly. The tibial knee component can be removed from the tibial knee component mold assembly, and the femoral knee component can be removed from the femoral knee component mold assembly, and the intramedullary canal filling component can be removed from the intramedullary canal filling component mold assembly, subsequent to desired curing of the flowable antibiotic laden bone cement within the relevant mold assembly. Removing the tibial knee component can comprise threading a threaded removal tool comprising an extending protrusion onto the threaded port of the tibial knee component mold assembly to push against and separate the tibial knee component from one of the mold members.

According to other features, the intramedullary canal filling component mold assembly can be overlayed on an x-ray and the flexible elongated member can be bent to match the actual anatomy of an intramedullary canal of a specific individual patient as shown on the x-ray during curing. The elongated intramedullary canal filling component mold cavity can comprise an elongated, cylindrical mold cavity and a reduced-dimension, axially extending cavity to form a grasping portion at a trailing end where the grasping portion can be grasped to remove the intramedullary canal filling component from an intramedullary canal of a patient. Bone cement in a doughy state can be placed between a trailing end of the intramedullary canal filling component removed from intramedullary canal filling component mold assembly and an adjacent one of the tibial knee component removed from the tibial knee component mold assembly or the femoral knee component removed from the femoral knee component mold assembly to couple the two components together. An intramedullary canal filling component formed from the intramedullary canal filling component mold assembly can be inserted into an intramedullary canal of a patient, and an adjacent one of the tibial knee component removed from the tibial knee component mold assembly or the femoral knee component removed from the femoral knee component mold assembly can be positioned with the intramedullary canal filling component in a relative anterior-posterior offset position, a relative lateral-medial position, or both to accommodate the actual anatomy of a specific individual patient.

A cement mold assembly configured to form a component of a temporary knee implant for use in delivering antibiotics to an infected site, the cement mold assembly is characterized by a first mold member having a first closed end, a second open end, and a sidewall defining a cavity. A second mold member has a base portion. The base portion has a footprint complementary to the sidewall of the first mold member such that the second mold member is slidably and progressively receivable by the sidewall into the cavity in a direction toward the closed end to define a tibial knee component forming cavity including a stem forming cavity and a platform forming cavity. Engagement members on the first mold member and the second mold member cooperate to enable progressive indexing of the first mold member relative to the second mold member until a desired thickness of the platform forming cavity is obtained.

According to other features, the first mold member can define the stem forming cavity and can include a tibial bearing forming surface, and the second mold member includes a tibial tray forming surface. Alternatively, the first mold member can include a tibial tray forming surface, and the second mold member includes a tibial bearing forming surface and a cannulated portion defining the stem forming cavity. A first plate and a second plate can be positioned outboard of the first and second molds, respectively, and at least two rods can be connected between the first and second plates to selectively and progressively index the first plate relative to the second plate.

Progressive advancement of the second mold member into the cavity of the first mold member can urge cement within the tibial component forming cavity out of a fill port in at least one of the first mold member and the second mold member. A reinforcement member can be included, which is configured to be placed at least partially within the cavity prior to progressive advancement of the second mold into the cavity, and which is configured to be molded into the unitary tibial component. The reinforcement member can comprise a first lateral portion received in the platform forming cavity and generally opposing the first closed end, and a second longitudinal portion that is received in the stem forming cavity.

According to other features, the base portion of the second mold can have a textured end surface. The first mold member can include gradations on the sidewall. A threaded inlet port for providing antibiotic laden bone cement into the tibial knee component forming cavity can be provided and a threaded removal tool including a protrusion can be configured to extend through the threaded port of the tibial knee component mold assembly to push against and separate the tibial knee component from one of the mold members as the threaded removal tool is threaded onto the inlet port.

A method of making a temporary knee implant for use in delivering antibiotics to an infected site is characterized by obtaining a first mold member having a first closed end, a second open end, and a sidewall defining a cavity. A second mold member can be obtained that has a base portion, and the base portion has a footprint complementary to the sidewall of the first mold member. The base portion of the second mold member is inserted into the cavity of the first mold member such that the second mold member is slidably and progressively receivable by the sidewall into the cavity in a direction toward the closed end to define an implant forming cavity including a stem forming cavity and a platform forming cavity. At least one of the first and second molds is advanced toward the other of the first and second molds by successively engaging cooperating engagement members of the first and second mold members. The advancing is ceased when a desired thickness of the platform forming cavity is obtained. Flowable material is inserted into the implant forming cavity. Subsequent to curing of the flowable material to a desired state, the temporary implant is removed from the first mold, and the temporary implant has an integrally formed tibial knee implant body and implant stem formed by the respective implant body forming cavity and the implant stem forming cavity.

According to other features, the implant forming cavity can comprise a tibial knee component forming cavity. The implant forming cavity can comprise a femoral component forming cavity. A flowable material delivery device can be coupled to a connection port into the implant forming cavity. Inserting flowable material into the implant forming cavity can occur prior to ceasing the advancing, and the advancing can cause flowable material to flow out a fill port of at least one of the first and second molds.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an exploded perspective view of a cement mold assembly constructed in accordance to one example of the present disclosure.
FIG. 2 is a perspective view of the cement mold assembly of FIG. 1 shown in an assembled position.
FIG. 3 is a cross-sectional view taken along lines 3-3 of FIG. 2.
FIG. 4 is a cross-sectional view taken along lines 4-4 of FIG. 2.
FIG. 5 is a cross-sectional view of the cement mold assembly shown subsequent to delivery of cement within the tibial component forming cavity.
FIG. 6 is an exploded bottom perspective view of a cement mold assembly constructed in accordance to additional features of the present disclosure.
FIG. 7 is a top perspective view of the cement mold assembly of FIG. 6 and further illustrating an optional reinforcement member configured to be molded as part of the resulting temporary implant.
FIG. 8 is a cross-sectional view of the cement mold assembly of FIG. 6.
FIG. 9 is another cross-sectional view of the cement mold assembly of FIG. 6.
FIG. 10 is an exploded perspective view of a cement mold assembly constructed in accordance to further features of the present disclosure.
FIG. 11 is a cross-sectional view taken along lines 11-11 of FIG. 10.
FIG. 12 is a cross-sectional view of the cement mold assembly of FIG. 11 shown subsequent to removing of a plug and introduction of flowable cement.
FIG. 13 is a perspective view of an alternative femoral component cement mold assembly constructed in accordance with features of the present disclosure.
FIG. 14 is a cross-sectional view of the femoral component cement mold assembly of FIG. 13.
FIG. 15 is a cross-sectional view of a intramedullary canal filling component cement mold assembly constructed in accordance with features of the present disclosure.
FIG 16 is a partial perspective view showing an end of a stem component molded with the mold of FIG. 15.
FIG. 17 is a perspective view of an alternative tibial component cement mold assembly constructed in accordance with features of the present disclosure.
FIG 17A is a perspective view of an implant removal tool, which is similar to the plug of FIG. 17.
FIG. 18 is a cross-sectional view of the tibial component cement mold assembly of FIG. 17.

Corresponding reference numerals indicate corresponding parts throughout the several views of the drawings.

### DETAILED DESCRIPTION

Example embodiments will now be described more fully with reference to the accompanying drawings.

The following description of the preferred embodiments concerning cement molds for temporary implants used during orthopedic surgical procedures are merely exemplary in nature and are not intended to limit the disclosure or its application or uses. Moreover, while the present invention is described in detail below with reference to cement molds for knee joints, it would be appreciated by those skilled in the art the present disclosure is clearly not limited to only cement molds for knee joints but may be utilized at various other areas for various other orthopedic surgical procedures. It will be understood that the terms "spacers" and "temporary implants" are used interchangeably throughout this disclosure to refer to the same component. A non-limiting discussion of terms and phrases intended to aid understanding of the present technology is provided at the end of this Detailed Description.

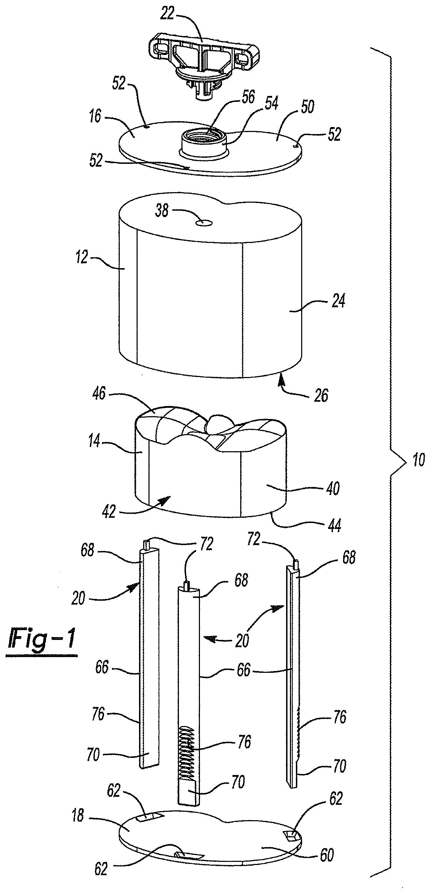

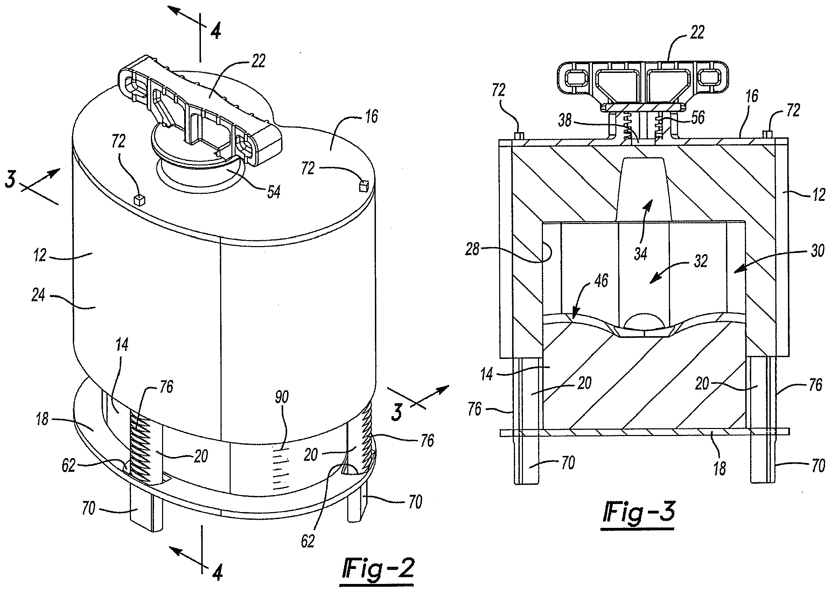

With initial reference to FIGS. 1-5, a cement mold assembly configured to form a temporary implant for use in delivering antibiotics to an infected site is shown and generally identified at reference numeral 10. The cement mold assembly 10 can generally include a first mold 12, a second mold 14, a first plate 16, a second plate 18, and a plurality of rods 20. The cement mold assembly 10 can further include a plug 22. As will become appreciated from the following discussion, the second mold 14 can be selectively and progressively advanced into the first mold 12 to create a unitary tibial component having a tibial tray portion and a stem portion.

The first mold 12 can generally include a first mold body 24 having an open end 26 and an inner wall 28. The first mold 12 can define a tibial component forming cavity 30 including a platform forming cavity 32 and a stem forming cavity 34. A first mold port 38 can be formed through the mold body 24 and aligned with the stem forming cavity 34.

The second mold 14 can include a second mold body portion 40 having an outer wall 42, an end surface 44 and a tibial platform forming surface 46. In one example, the tibial platform forming surface 46 can include contours configured to substantially replicate a corresponding tibial bearing surface. The dimensions of the outer wall 42 of the second mold body portion 40 are slightly less than the inner wall 28 of the first mold 12 such that the second mold 14 can be selectively and progressively advanced into the tibial component forming cavity 30. In one example, the first and second molds 12 and 14 can be formed of silicone.

The first plate 16 can include a first plate body portion 50 having a plurality of apertures 52. A first plate boss 54 can extend from the first plate 16. The first plate boss 54 can include a threaded delivery port 56. The second plate 18 can include a second plate body portion 60 having a plurality of apertures 62. Each of the rods 20 can generally be formed by an elongated body portion 66 having first ends 68 and second ends 70. A projection 72 can be formed at a distal tip of each of the first ends 68.

Teeth 76 can be formed along each of the elongated body portion 66. The second ends 70 can be received into the respective apertures 62 on the second plate 18. The projections 72 on the first ends 68 of the rods 20 can be received by the apertures 52 in the first plate 16. As will become appreciated herein, the teeth 76 can progressively engage the second plate body portion 60 at the respective apertures 62 upon progressive advancement of the second plate 18 toward the first plate 16 (resulting in the second mold 14 being progressively received within the first mold 12).

It will be appreciated that continued advancement of the second mold 14 within the tibial component forming cavity 30 will control the resulting height or thickness of the resulting tibial platform formed by the tibial platform forming cavity 32. The thickness can be monitored such as by gradations 90 provided on the outer wall 42 of the second mold 14. The gradations 90 may be provided on other components of the cement mold assembly 10. Additionally or alternatively, a surgeon can deduce the thickness of the resulting tibial tray platform by the amount of teeth 76 that have been advanced through the respective aperture 62 on the second plate.

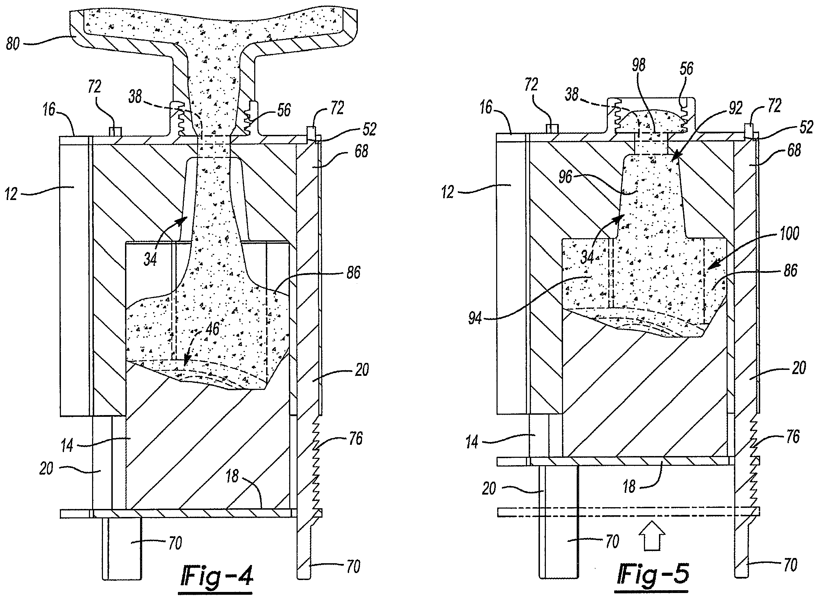

With particular reference now to FIGS. 4 and 5, a method of making a temporary implant for use in delivering antibiotics to an infected site using the mold assembly 10 will be described in greater detail. In one example, the surgeon can progressively advance the second mold 14 into the tibial component forming cavity 30 of the first mold 12 to a desired depth or cavity thickness. Then, the surgeon can couple a flowable material delivery device 80 to the threaded delivery port 56 on the first plate 16. In one example, the flowable material delivery device 80 can include a handle (not specifically shown) that can be actuated to influence emission of flowable material 86 out of the delivery device 80 to fill the tibial component forming cavity 30. Other configurations of the flowable material delivery device may be used. Furthermore, it will be appreciated that other connections may be made between the flowable material delivery device 80 and the respective port 38. For example, a luer lock coupling, a nozzle coupling or other coupling may be employed.

Once a sufficient amount of flowable material 86 has been advanced into the tibial component forming cavity 30, the flowable material delivery device 80 may be removed. Plug 22 can then be threaded onto the threaded delivery port 56 and the flowable antibiotic laden cement allowed to harden to a desired state. Thus, a partial overview of this process involves assembling the mold assembly 10, then progressively moving the first mold 12 and second mold 14 toward each other to achieve a desired or selected depth or thickness of the tibial component forming cavity 30, then filling the tibial component forming cavity 30 with flowable material and allowing it to harden sufficiently, and then removing the tibial component from the mold assembly 10.

Once the flowable material 86 has cured a sufficient amount, the mold assembly 10 can be removed from a resulting temporary implant 92. The resulting temporary implant 92 can include a tibial tray portion 94 and a stem portion 96. A portion 98 of cured flowable material 86 shown above the stem portion 96 may be discarded. The mold assembly 10 can be removed by any process such as by cutting away the first mold 12. Additionally or alternatively, a surgeon can retract the second mold 14 out of the tibial component forming cavity 30. Other methods are contemplated.

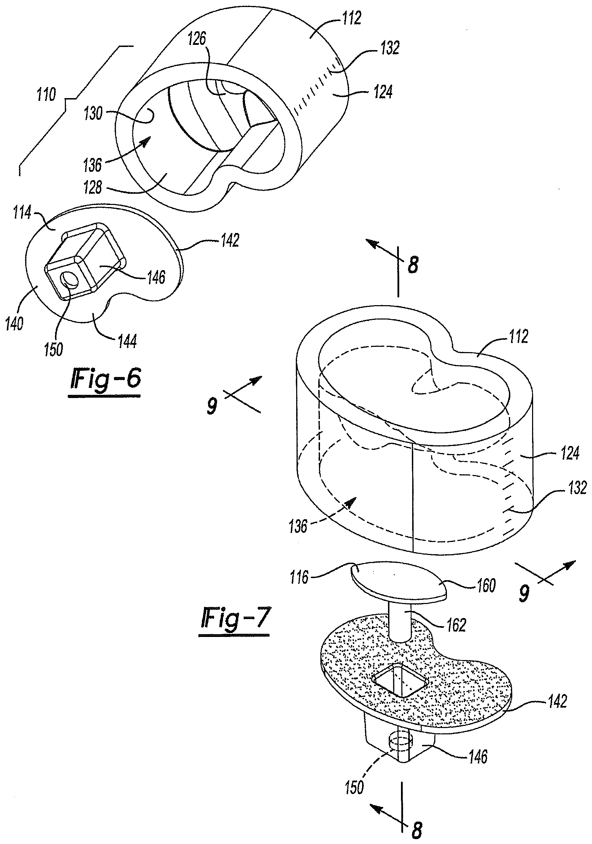

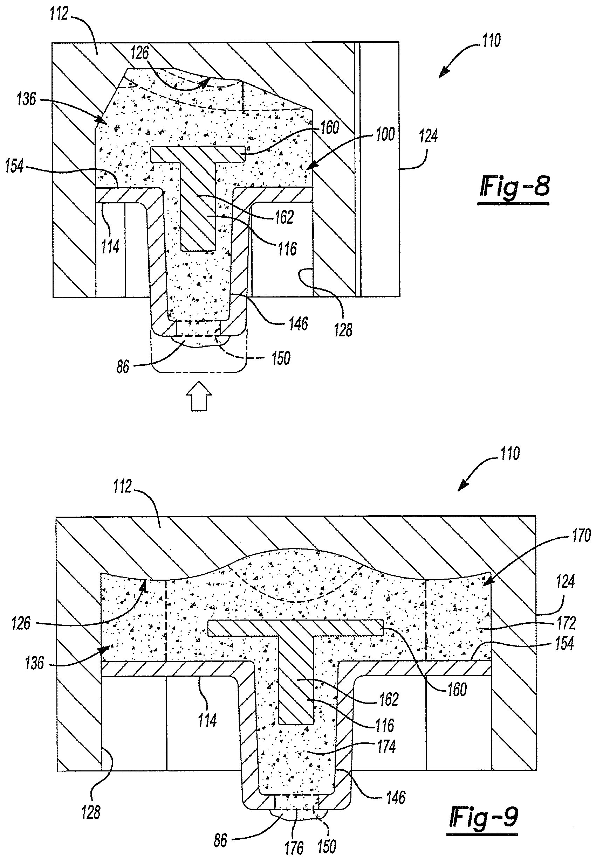

With reference now to FIGS. 6-9, a cement mold assembly configured to form a temporary implant for use in delivering antibiotics to an infected site constructed in accordance to additional features of the disclosure is shown and generally identified at reference numeral 110. The cement mold assembly 110 can generally include a first mold 112 and a second mold 114. According to one example, the mold assembly 110 can further incorporate an optional reinforcing member 116 (shown in FIGS. 7-9). As will become appreciated from the following discussion, the reinforcing member 116 can be used with the mold assembly 110 in instances where a solid reinforcing structure is to be molded within the temporary implant. As will be disclosed herein, the second mold 114 can be selectively and progressively advanced into the first mold 112 to create a unitary tibial component having a tibial tray portion and a stem portion.

The first mold 112 can generally include a mold body 124 having a first closed end 126 and a second open end 128. The mold body 124 can include a sidewall 130. The sidewall 130 can include gradations 132 formed thereon. The first mold 112 can define a cavity 136. The first mold 112 can be formed of transparent or semi-transparent material to observe formation of the temporary implant. Any of the other molds disclosed herein may also be formed of transparent or semi-transparent material.

The second mold 114 can include a second mold body 140 having an outer wall 142. The second mold 114 can further include a base portion 144 and a cannulated member 146 extending therefrom. The base portion 144 can have a footprint formed by the wall 142 that is complementary to the geometry of the sidewall 130 of the first mold 112. As will become appreciated from the following discussion, the second mold 114 can be slidably and progressively received by the sidewall 130 into the cavity 136 in a direction toward the first closed end 126. The cannulated member 146 can further define an inlet port 150 defined therethrough. In the example provided, the cannulated member 146 can have a generally rectangular cross-section. The base portion 144 of the second mold 114 can include a textured end surface 154. In one example, the textured end surface 154 can facilitate removal of the resulting tibial tray from the mold assembly 110 subsequent to formation. The first mold 112 can be formed of silicone. The second mold 114 can be formed of polyethylene.

The reinforcement member 116 can include a first lateral portion 160 that can generally oppose the first closed end 126 and a second longitudinal portion 162 that is generally received by the cannulated member 146. The reinforcement member 116 can be configured to be placed at least partially within the cavity 136 prior to progressive advancement of the second mold 114 into the cavity 136. The reinforcement member 116 can be configured to be molded into the resulting unitary tibial component. It will be appreciated that the geometry of the reinforcement member 116 is merely exemplary. In this regard, the reinforcement member 116 may be shaped in any configuration. Moreover, any of the temporary implants disclosed herein may be molded with a reinforcement member therein. The reinforcement member 116 can be formed of biocompatible metal.

An exemplary method of using the mold assembly 110 according to one example will now be described. While not specifically shown, a flowable material delivery device such as described above may be coupled at the port 150 to inject flowable material into the cavity 136 between the first and second molds 112 and 114. Once a sufficient amount of flowable material 86 has been advanced into the tibial component forming cavity 136, the flowable material delivery device may be removed. Next, a surgeon can advance the second mold 114 toward the first mold 112 causing the second mold 114 to be progressively advanced into the tibial component forming cavity 136 of the first mold 112. It will be appreciated that continued advancement of the second mold 114 within the tibial component forming cavity 136 will control the resulting height or thickness of the resulting tibial platform formed by the tibial platform forming cavity 172. The thickness can be monitored such as by gradations 132 provided on the inner or outer wall 124 surface of the second mold 114. The gradations 132 may be provided on other components of the cement mold assembly 110. Additionally or alternatively, a surgeon can deduce the thickness of the resulting depth of the tibial tray cavity 172 by the depth of cavity 136 visible above the second mold 114. It will further be appreciated that during such advancement, excess flowable material 86 will be expelled through the port 150 and out of the tibial component forming cavity 30.

The second mold 114 can be advanced toward the first closed end 126 of the first mold 112 until the desired tibial tray thickness has been attained. It will be appreciated that additionally or alternatively the first mold 112 may be advanced toward the second mold 114. In some examples, a surgeon can reference the gradations 132 on the sidewall 130 of the first mold 112. The gradations 132 can be formed on the outer surface or inner surface of the sidewall 130. It will be appreciated that in some examples, excess flowable material may be expelled through the port 150.

Once the flowable material has cured a sufficient amount, the mold assembly 110 can be removed from a resulting temporary implant 170. The resulting temporary implant 170 can include a tibial tray portion 172 and a stem portion 174. A portion 176 of cured flowable material may be discarded. As identified above, the mold assembly 110 can be removed by any process.

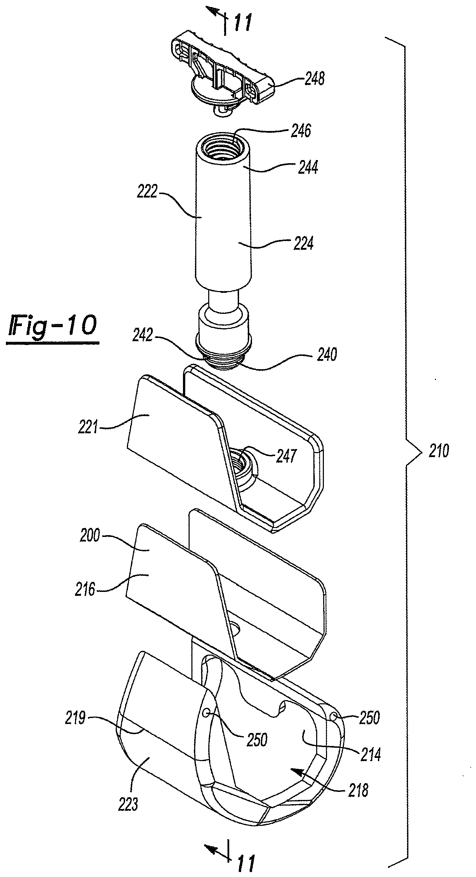

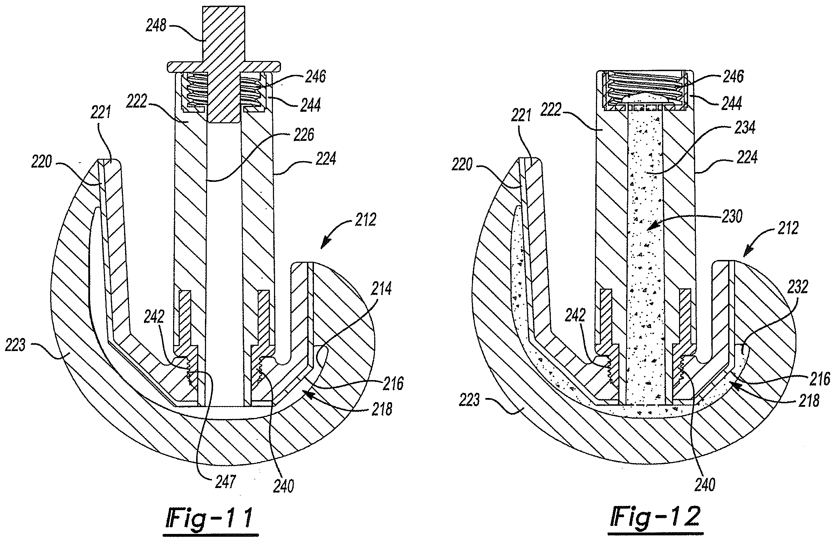

With reference now to FIGS. 10-12, a cement mold assembly configured to form a temporary implant for use in delivering antibiotics to an infected site according to other features is shown and generally identified at reference numeral 210. The cement mold assembly 210 can include a mold body 212 (FIG. 11) having an outer sidewall 214 and an inner sidewall 216 that cooperate to define a cavity 218. The inner sidewall 216 can be operable to define a shape of the temporary implant.

The mold body 212 can be collectively formed by a first mold member 219, a second mold member 220, a third mold member 221 and a fourth mold member 222. The mold body 212 can include an articulating forming portion 223 and a stem forming portion 224. The cavity 218 is collectively defined by the articulating forming portion 223 and the stem forming portion 224. Specifically, the stem forming portion 224 includes an inner surface 226 that cooperates with the inner sidewall 216 in an assembled position (FIG. 11). The articulating forming portion 223 and the stem forming portion 224 can cooperate to form a unitary femoral component 230 (FIG. 12) having an articulating portion 232 formed by the articulating forming portion 223 and a stem portion 234 formed by the stem forming portion 224.

In one example, the stem forming portion 224 can be coupled to the articulating forming portion 223. In the example shown, the stem forming portion 224 is threadably coupled to the articulating forming portion 223. The stem forming portion 224 can have a first end 240 defining threads 242 and a second end 244 defining second threads 246. The first threads 242 can be configured to threadably couple with threads 247 on the third mold member 221. The second threads 246 can be configured to threadably couple with plug 248 or a cement delivery device (not shown). Flowable material may be injected into the cavity 218 similar to that described above. In one example, the mold body 212 can define vents 250 configured to facilitate escaping air during delivery of the flowable material. It will be appreciated that vents may be additionally incorporated on any of the other molds disclosed herein. A reinforcement member, similar to the reinforcement member 116 (FIG. 7) can be optionally included as part of the cement mold assembly 210 for molding into the resulting unitary femoral component. The unitary femoral component 230 may be removed from the cement mold assembly 210.

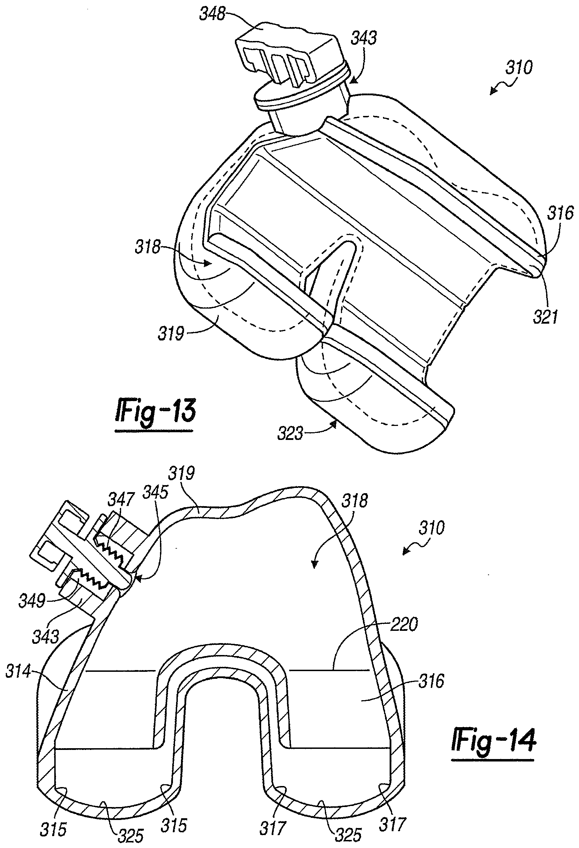

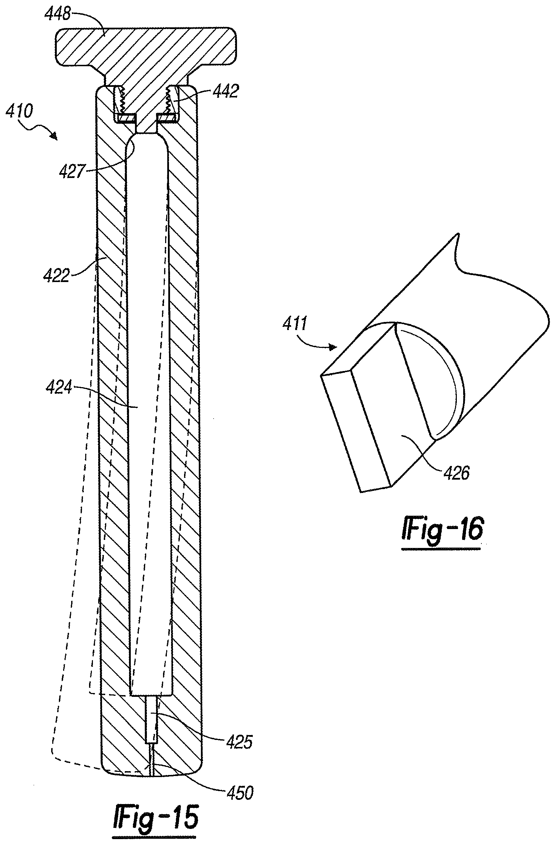

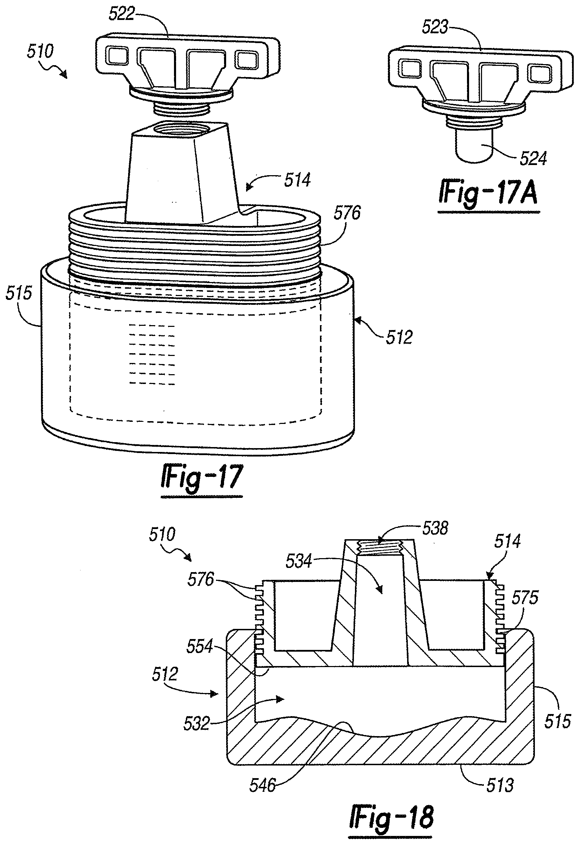

FIGS. 13-18 illustrate additional examples of various cement mold assemblies for producing antibiotic loaded cement molded components for a temporary knee joint spacer. Thus, as with the above examples, the cement mold assembly 310 of FIGS. 13 and 14 for forming the femoral knee joint spacer component, the cement mold assembly 410 of FIG 15 for forming a separate intramedullary canal filling component 411 of FIG 16, and the cement mold assembly 510 of FIGS. 17 and 18 for forming a temporary tibial component, can be provided separately or together (for example, in a kit) and can be used together in order to provide all desired antibiotic cement-molded components for a temporary knee joint spacer.

FIGS. 13 and 14 illustrate the femoral component cement mold assembly 310. This example is similar to the femoral component portion of the mold assembly 210 illustrated in FIGS. 10-12 and previously described herein. The primary differences between this femoral component mold assembly example 310 and the previously described mold assembly 210 is the cement is filled through a port or opening 345 of coupling 343 in the first mold member 319, which is the articulating forming portion 323, and this mold assembly 310 does not include any mold components for forming an integrally molded stem.

The coupling 343 is included on, and provides a port or opening 345 through, the first mold member 319, which can be made of silicone. Thus, antibiotic laden bone cement can be provided to the cavity 318 through the opening 345. The opening 345 provided in the first mold member 319 can be provided along a side so that the opening 345 is outside or away from the primary articulating surface of the molded femoral component that engages against the molded tibial component. In such a case, for example, the opening 345 into the cavity 318 would not be provided in the outer wall 314 between corners 315 and corners 317 of outer wall 314.

As seen in this example, the opening 345 intersects the cavity 318 at an angle and on the side of wall 314 between the primary tibial spacer contacting articulating surface-forming wall 325 and the femoral bone engaging or directly opposing forming wall (inner surface of inner sidewall member 316). As such, the opening 345 can be located in a substantially lateral position of the articulating surface, or - stated another way - lateral of the primary articulating surface. The opening can additionally be located in an anterior position of the articulating surface, or - stated another way - anterior of the primary articulating surface of the molded femoral component that engages against the molded tibial component.

As with the femoral component forming mold assembly 210 example of FIGS. 10-12, the bone engaging or directly opposing surface forming walls can include an inner sidewall silicone member 316 coupled against a rigid mold member 321. The outer rigid member 321 can be formed, for example, of a polycarbonate, a polyamide, or a copolyester. The inner sidewall member 316 can be made of silicone and can include vents in the corners (like vents 250 of Fig. 10) to insure complete filling of the mold cavity 318. After the mold cavity 318 is completely filled, the plug 348 can be coupled to the coupling 343 via cooperating threads 347 to seal the fill opening 345. For example, a silicone first mold member 319 can include a threaded insert of more rigid material carrying the threads 347 to cooperate with the threads 347 of the plug 348.

FIGS. 17 and 18 illustrate a cement mold assembly for forming a temporary tibial spacer component 510. This example of a tibial spacer cement mold assembly 510 can include a first mold 512, a second mold 514 that is received in the first mold 512, and a plug 522 for sealing fill port or opening 538 in the second mold member 514. In this example, the first mold member 512 includes a tibial platform forming surface 546, which forms the upper surface of the molded tibial component that engages the articulating surface of a cooperating femoral component formed using the femoral mold assembly 310.

The first mold member 512 includes inwardly projecting engagement members 575. These engagement members 575 are configured to engage cooperating engagement members 576 of the second mold member 514 as the second mold 514 is pushed into the first mold member 512 similar to that already described above with regard to the examples of FIGS. 1-5. The engagement members 575 and cooperating engagement members 576 can have a variety of shapes. As illustrated, the engagement members 575 and cooperating engagement members 576 can each include one or more projecting members that successively engage within one or more grooves between the cooperating projecting members.

In addition, the various inter-engagement members 575 and 576 and resulting grooves therebetween can have a squared configuration as seen in FIG. 18, or can have an angled or serrated configuration (similar to the teeth 76 shown in FIGS. 4 and 5). In the latter case, for example, the engagement members 575 and 576 could be angled upward (in the orientation shown in FIGS. 17 and 18) on the inner mold member 514 and angled downward on the outer mold member 512. The engagement members 575 and 576 can extend around the entire inner periphery of the first mold member 514, or can be interrupted. For example, three, four, or more separated vertical (as illustrated) regions equally spaced around the periphery can be provided with the cooperating engagement members 575 and 576. This is similar to the three rods 66, each providing one of three regions of teeth 76 around the periphery as shown, for example, in FIG. 1.

The second mold member 514 cooperates with the first mold member 512 to form a stem-forming cavity 534 and a platform-forming cavity 532. This is similar to that illustrated and described in reference to FIGS. 1-7. Also, the internal surface 554 of the second mold member 514 can be textured similar to that illustrated and described in reference to FIG. 7. The second mold member 514 can be made of any suitable material. For example, polyethylene or polypropylene can be used to form the second mold member 514.

The cooperating engagement members 576 of the second mold member 514 can be in the form of peripheral protrusions, valleys, or both. Thus, the cooperating engagement members 576 can be configured similarly to that of the engagement members 575 described above. Thus, there can be only one or more horizontal (as illustrated) level of engagement member(s) 576, and can extend continuously around the entire outer periphery, or can be discontinuous such that they are located in three, four, or more equally spaced vertical regions.

The second mold member 514 can have a threaded opening 538 providing a fill opening or passage into the mold cavity 532 and 534. Upon filling of the mold cavity 532 and 534, the plug 522 can be threaded onto the second mold member 514 to seal the fill opening 538. It should be appreciated that the tibial component mold assembly 510 of FIGS. 17 and 18, can be employed in processes that are essentially the same as those described with regard to the example of the tibial component mold assembly 10 of FIGS. 1-5 and with regard to the tibial component mold assembly 110 of Figs. 6 and 7.

After the flowable material has hardened to the desired state, the surgeon can cut the first mold member 512, or otherwise separate the first mold member 512 from the second mold member 514. This can leave the molded tibial knee component attached to the second mold member 514. This can occur in any case, but especially when the surface 554 of the second mold member 514 is textured. Plug 522 as illustrated in FIG. 17 can be removed from the threaded opening 538 and replaced with the removal tool 523 illustrated in FIG. 17A. As the removal tool 523 is threaded into the threaded opening 538, the extending protrusion 524 pushes against the adjacent surface of the tibial knee implant to push and separate it from the second mold member 514.

It should be appreciated that the nose or extending protrusion 524 has been exaggerated in the drawings and/or the threads have been shortened to emphasize the protrusion 524. In actuality, however, the cooperating threads on threaded opening 538 and the removal tool 523 will engage each other prior to the distal end of the extending protrusion 524 contacting the molded tibial implant. Thus, as the removal tool 523 continues to be threaded into the threaded opening 538, the extending protrusion 524 engages and then pushes against the adjacent surface of the tibial knee implant to push and separate it from the second mold member 514. In one example the length of the nose or extending protrusion 524 can be about 5 mm. The length of the threads of threaded opening 538, of the removal tool 523, or both can be greater than 5 mm.

FIG. 15 illustrates a separate intramedullary canal filling component mold assembly 410 and FIG. 16 partially illustrates an intramedullary canal filling component 411 molded therefrom. In some cases, there is a desire to provide a temporary intramedullary canal implant 411 formed from antibiotic-laden bone cement into an elongated cavity previously formed into the bone to accommodate a stem extending from a tibial knee component or a femoral knee component which has been removed. This might occur, for example, if an infection extends into such an intramedullary canal. The intramedullary canal filling component mold assembly 410 can prepare a separate intramedullary canal filling component 411 for such purpose.

The mold assembly 410 can include an elongated mold member 422 that can provide an elongated, cylindrical mold cavity 424. One end of the elongated cavity 424 can provide a reduced-dimension, axially extending tab-forming cavity 425. The reduced dimension tab forming cavity 425, can be configured to form a plate member 426 on one end of the molded temporary intramedullary canal implant 411 as shown in FIG. 16. A radius can be provided at the interface of the base of the plate member 426 and the end surface of the cylindrical portion of the intramedullary canal filling component 411 to help manage stresses which might otherwise cause the plate member 426 to separate therefrom. In this way, the reduced-dimension axially extending tab 426 provides an axially extending portion that can be readily grasped by a surgeon to remove the intramedullary canal implant 411 from an elongated bone cavity during a subsequent surgery.

The opposite end of the elongated mold cavity 424 can include a radiused surface 427. The radiused surface 427 can be configured to make it easier to insert the intramedullary canal filling component 411 molded from the mold assembly 410, radiused end first, into an elongated stem cavity in a bone. The mold cavity 424 can be filled through a threaded port or opening 442, which can be sealed by a threaded plug 448 after filling. In order to facilitate complete filling of the intramedullary canal filling component forming cavity 424, a vent port 450 can be provided at the end of the intramedullary canal filling component forming mold cavity 424 that is opposite the fill opening 442.

The first mold member 422 can be made from a flexible material. In this way the first mold member 422 can be angled or bent at virtually any point along its length. In fact, it can be angled or bent at a plurality of places along the length (as indicated by the dotted lines in FIG. 15. For example, the first mold member 422 can be bent to provide a intramedullary canal filling component forming cavity 424 with one or more relatively straight sections at an angle relative to one or more angled sections in order to accommodate for any variations in a preexisting bone cavity. In such a case, the first mold member 422 can be bent into an appropriate configuration or position and held in such bent position during curing of the antibiotic laden bone cement within the mold cavity 424. Such flexibility can allow the first mold member 422 to be bent to match an x-ray, for example, by laying it directly on an x-ray during curing.

After set-up of the antibiotic-laden bone cement in the mold cavity 424, the surgeon can simply cut or sever mold member 422 to remove the temporary intramedullary canal implant from the mold cavity 424. Alternatively, the first mold member 422 can comprise two sub mold members (not shown) that can be threaded together near the threaded opening 442, for example. Such sub members can simply be unscrewed in order to facilitate removal of the molded intramedullary canal filling component 411 from the mold cavity 424.

To accommodate for preexisting elongated intramedullary canals in a bone having different lengths, the mold member 422 can potentially be cut to the desired length. The cut end can be cut or otherwise shaped to have a radiused surface at its insertion end. For example, the mold 422 and cement can be cut by a surgeon before the cement has fully cured or hardened to facilitate cutting and any reshaping, or the molded intramedullary canal implant can be cut after it has been removed from the mold assembly 410.

Alternatively, a manufacturer can offer or otherwise provide a plurality of first mold members 422 or separate intramedullary canal filling component assemblies 410, each having a different axial length. Thus, a surgeon can simply choose the mold member 422 or mold assembly 422 providing the most appropriate mold cavity 424 axial length.

After removal of a molded intramedullary canal filling component 411 from the mold assembly 410, the temporary separate molded intramedullary canal filling component 411 can be inserted into the intramedullary canal. The radiused end can be inserted first. This makes the reduced dimension grasping tab 426 available for use in facilitating insertion of the separate temporary intramedullary canal filling component into the preexisting intramedullary canal bone cavity. At a point in time when it is desirable to remove the temporary intramedullary canal filling component 411, a surgeon can use the reduced-dimension grasping tab 426 to facilitate removal of the temporary intramedullary canal implant 411. For example, a surgeon can grasp the tab 426 with a surgical instrument such as pliers to pull the temporary intramedullary canal implant 411 from the intramedullary canal bone cavity.

Alternatively, the intramedullary canal implant 411 can be joined to a molded tibial component during the implantation surgery. For example, a surgeon can place antibiotic laden bone cement that is in a doughy state around plate member 426 of intramedullary canal implant 411. Upon hardening, this doughy cement can couple the intramedullary canal implant 411 to the adjacent implant, such as that formed by the mold assembly 310 or the mold assembly 510. Thus, during surgery to remove the temporary spacer implant components, the intramedullary canal implant 411 can be pulled out of the intramedullary canal along with the removal of the adjacent implant to which it has been coupled (forming a single piece integral component) during the prior implant surgery.

Providing a separate mold assembly 410 for molding a separate intramedullary canal implant 411 as described herein with reference to FIGS. 15 and 16 can permit not only bending of the first mold member 422 to match an x-ray showing the actual anatomy of the intramedullary canal of a specific individual patient, but also can permit positioning of the molded intramedullary canal implant 411 relative to the adjacent spacer implant component to match the actual anterior-posterior positioning, and the actual lateral-medial positioning of the intramedullary canal relative to the position of the adjacent implant component, which corresponds to the actual anatomy of the specific individual patient.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications may be within the scope of the invention, which is defined by the appended claims.

### Non-limiting Discussion of Terminology

The headings (such as "Introduction" and "Summary") and subheadings used herein are intended only for general organization of topics within the present disclosure, and are not intended to limit the disclosure of the technology or any aspect thereof. In particular, subject matter disclosed in the "Introduction" may include novel technology and may not constitute a recitation of prior art. Subject matter disclosed in the "Summary" is not an exhaustive or complete disclosure of the entire scope of the technology or any embodiments thereof. Classification or discussion of a material within a section of this specification as having a particular utility is made for convenience, and no inference should be drawn that the material must necessarily or solely function in accordance with its classification herein when it is used in any given composition.

The description and specific examples, while indicating embodiments of the technology, are intended for purposes of illustration only and are not intended to limit the scope of the technology. Moreover, recitation of multiple embodiments having stated features is not intended to exclude other embodiments having additional features, or other embodiments incorporating different combinations of the stated features. Specific examples are provided for illustrative purposes of how to make and use the compositions and methods of this technology and, unless explicitly stated otherwise, are not intended to be a representation that given embodiments of this technology have, or have not, been made or tested.

As used herein, the words "desire" or "desirable" refer to embodiments of the technology that afford certain benefits, under certain circumstances. However, other embodiments may also be desirable, under the same or other circumstances. Furthermore, the recitation of one or more desired embodiments does not imply that other embodiments are not useful, and is not intended to exclude other embodiments from the scope of the technology.

As used herein, the word "include," and its variants, is intended to be non-limiting, such that recitation of items in a list is not to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments of the present technology that do not contain those elements or features.

Although the open-ended term "comprising," as a synonym of non- restrictive terms such as including, containing, or having, is used herein to describe and claim embodiments of the present technology, embodiments may alternatively be described using more limiting terms such as "consisting of or "consisting essentially of." Thus, for any given embodiment reciting materials, components or process steps, the present technology also specifically includes embodiments consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

When an element or layer is referred to as being "on", "engaged to", "connected to" or "coupled to" another element or layer, it may be directly on, engaged, connected or coupled to the other element or layer, or intervening elements or layers may be present. In contrast, when an element is referred to as being "directly on", "directly engaged to", "directly connected to" or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

## Claims

1. A combination of three separate cement mold assemblies (10; 110; 210; 310; 410; 510), each configured to form a component of a temporary knee implant for use in delivering antibiotics to an infected site of a knee, the combination comprising:
a tibial knee component mold assembly (10; 110; 510), the tibial knee component mold assembly having a second mold member (14; 114; 514) receivable within a first mold member (12; 112; 512) to define a tibial component mold cavity (30; 136; 532) therebetween, the tibial component mold cavity (30; 136; 532) defining a tibial tray surface and a tibial bearing surface and optionally a stem portion, and the second mold member (14; 114; 514) being configured to be selectively and progressively indexed relative to the first mold member (12; 112; 512) via cooperating engagement members (76; 575, 576) until a desired thickness of the tibial component mold cavity (30) is obtained;
a femoral knee component mold assembly (210; 310), the femoral knee component mold assembly (210; 310) having a femoral component mold cavity (218; 318) formed between an inner sidewall (216; 316) defining a shape of a bone opposing surface and an outer side wall (214; 314) defining a shape of an articulating surface which contacts a tibial tray of a tibial component;
the combination being **characterized by**
an intramedullary canal filling component mold assembly (410), the intramedullary canal filling component mold assembly (410) having an elongated intramedullary canal filling component mold cavity (424, 425) defined by a flexible elongated member (422) enabling adjustment of the mold cavity (424, 425) to correspond to an intramedullary canal of an individual patient.

2. The combination of cement mold assemblies according claim 1, **characterized in that** the tibial bone opposing surface of the tibial component mold assembly (110; 510) comprises a textured surface (154; 554).

3. The combination of cement mold assemblies according any of claims 1 and 2, **characterized in that** the one of the first mold member (12; 112) and the second mold member (14; 114) of the tibial component mold assembly (10; 110) comprise gradations (90; 132).

4. The combination of cement mold assemblies according to any of claims 1 - 3, **characterized in that** the inner sidewall (216; 316) comprises an inner layer comprising silicone coupled to an outer layer comprising a material that is more rigid than silicone, and optionally the rigid material is one of a polycarbonate, a polyamide, and a copolyester.

5. The combination of cement mold assemblies according to any of claims 1 - 4, **characterized by** a coupling (343) providing a fill port (345) into the femoral component mold cavity (318) at a position that is lateral of a portion of the articulating surface which primarily contacts a tibial tray of a tibial component, and is optionally lateral of the portion of the articulating surface which primarily contacts the tibial tray of the tibial component.

6. The combination of cement mold assemblies according to any of claims 1 - 5, **characterized in that** the elongated intramedullary canal filling component mold cavity (424, 425) of the intramedullary canal filling component mold assembly comprises an elongated, cylindrical mold cavity (424) and a reduced-dimension, axially extending cavity (425) at one end.

7. The combination of cement mold assemblies according to claim 6, **characterized in that** the intramedullary canal filling component mold assembly (410) comprises a radiused surface (427) at an end opposite the one end to facilitate insertion of a molded stem component in an elongated bone cavity.

8. The combination of cement mold assemblies according to any of claims 1 - 7, **characterized in that** the flexible elongated member of the intramedullary canal filling component mold assembly (410) is at least semi-transparent permitting an x-ray to be viewed through the flexible elongated member.

9. A method of making a temporary knee implant, the method comprising the steps of:
obtaining a tibial knee component mold assembly (10; 110; 510), the tibial knee component mold assembly (10; 110; 510) having a first mold member (12; 112; 512) and a second mold member (14; 114; 514);
inserting the second mold member (14; 114; 514) into the first mold member (12; 112; 512) to define a tibial knee component mold cavity (30; 136; 532) including a tibial platform forming portion, and optionally a stem forming portion;
advancing at least one of the first and second mold members (12, 14; 112, 114; 512, 514) toward the other of the first and second mold member (12, 14; 112, 114; 512, 514) by successively engaging cooperating engagement members (76; 575, 576) of the first and second mold members (12, 14; 112, 114; 512, 514) until a desired thickness of the platform forming portion is obtained;
obtaining a femoral knee component mold assembly (210; 310), the femoral knee component mold assembly (210; 310) having a femoral component mold cavity (218; 318) formed between an inner sidewall (216; 316) defining a shape of a bone opposing surface and an outer side wall (214; 314) defining a shape of an articulating surface for contacting a tibial tray of a tibial knee component; and the method being **characterized by** the step of:
obtaining a separate intramedullary canal filling component mold assembly (410), the intramedullary canal filling component mold assembly (410) having an elongated intramedullary canal filling component mold cavity (424, 425) defined by a flexible elongated member (422) enabling adjustment of the mold cavity (424, 425) to correspond to an intramedullary canal of an individual patient.

10. The method of claim 9, **characterized by**:
inserting flowable antibiotic laden bone cement into each of the tibial knee component mold cavity, the femoral component mold cavity, and the intramedullary canal filling component mold cavity through a port (38; 150; 345; 442; 538) in each of the respective tibial knee component mold assembly (10; 110; 510), femoral component mold assembly (210, 310), and intramedullary canal filling component mold assembly (410),
removing the tibial knee component from the tibial knee component mold assembly (10; 110; 510), and the femoral knee component from the femoral knee component mold assembly (210, 310), and the intramedullary canal filling component from the intramedullary canal filling component mold assembly (410), subsequent to desired curing of the flowable antibiotic laden bone cement within the relevant mold assembly (10; 110; 210; 310; 410; 510).

11. The method of claim 10, **characterized in that** removing the tibial knee component comprises threading a threaded removal tool (523) comprising an extending protrusion (524) onto the threaded port (538) of the tibial knee component mold assembly (510) to push against and separate the tibial knee component from one of the mold members (512; 514).

12. The method of any of claims 9 and 11, **characterized by** overlaying the intramedullary canal filling component mold assembly (410) on an x-ray and bending the flexible elongated member (422) to match the actual anatomy of an intramedullary canal of a specific individual patient as shown on the x-ray during curing.

13. The method of any of claims 9 - 12, **characterized in that** the elongated intramedullary canal filling component mold cavity (424, 425) comprises an elongated, cylindrical mold cavity (424) and a reduced-dimension, axially extending cavity (425) forms a grasping portion at a trailing end where the grasping portion can be grasped to remove the intramedullary canal filling component from an intramedullary canal of a patient.

14. The method of any of claims 9 - 13, **characterized by** placing bone cement in a doughy state between a trailing end of the intramedullary canal filling component removed from intramedullary canal filling component mold assembly (410) and an adjacent one of the tibial knee component removed from the tibial knee component mold assembly (10; 110; 510) or the femoral knee component removed from the femoral knee component mold assembly (210; 310).

## Patentansprüche

1. Kombination aus drei separaten Zementformbaugruppen (10; 110; 210; 310; 410; 510), die jeweils derart konfiguriert sind, eine Komponente eines temporären Knieimplantats zur Verwendung bei der Lieferung von Antibiotika an eine infizierte Stelle eines Knies zu bilden, wobei die Kombination umfasst:
eine Formbaugruppe (10; 110; 510) für eine Tibiakniekomponente, wobei die Formbaugruppe für die Tibiakniekomponente ein zweites Formelement (14; 114; 514) aufweist, das in einem ersten Formelement (12; 112; 512) aufnehmbar ist, um einen Tibiakomponentenformhohlraum (30; 136; 532) dazwischen zu definieren, wobei der Tibiakomponentenformhohlraum (30; 136; 532) eine Tibiaschalenfläche und eine Tibialagerfläche und optional einen Schaftabschnitt definiert, und das zweite Formelement (14; 114; 514) derart konfiguriert ist, dass es selektiv und progressiv relativ zu dem ersten Formelement (12; 112; 512) über zusammenwirkende Eingriffselemente (76; 575; 576) indexiert ist, bis eine gewünschte Dicke des Tibia-Komponentenformhohlraums (30) erhalten ist;
eine Formbaugruppe (210; 310) für die Femurkniekomponente, wobei die Formbaugruppe (210; 310) für die Femurkniekomponente einen Femurkomponentenformhohlraum (18, 318) aufweist, der zwischen einer inneren Seitenwand (216; 316), die eine Form einer Knochengegenfläche definiert, und einer Außenseitenwand (214; 314) gebildet ist, die eine Form einer Gelenkfläche definiert, die mit einer Tibiaschale einer Tibiakomponente in Kontakt steht;
wobei die Kombination **gekennzeichnet ist durch**:
eine Formbaugruppe (410) für eine einen intramedulären Kanal füllende Komponente, wobei die Formbaugruppe (410) für eine einen intramedulären Kanal füllende Komponente einen länglichen Formhohlraum (424, 425) für die den intramedulären Kanal füllende Komponente aufweist, der **durch** ein flexibles längliches Element (422) definiert ist, das ermöglicht, dass der Formhohlraum (424, 425) so eingestellt werden kann, dass er einem intramedulären Kanal eines einzelnen Patienten entspricht.

2. Kombination von Zementformbaugruppen nach Anspruch 1, **dadurch gekennzeichnet, dass** die dem Tibiaknochen gegenüberliegende Fläche der Tibiakomponentenformbaugruppe (110; 510) eine texturierte Fläche (154; 554) umfasst.

3. Kombination von Zementformbaugruppen nach einem der Ansprüche 1 oder 2, **dadurch gekennzeichnet, dass** das eine von dem ersten Formelement (12; 112) und dem zweiten Formelement (14; 114) der Tibiakomponentenformbaugruppe (10; 110) Abstufungen (90; 132) umfasst.

4. Kombination von Zementformbaugruppen nach einem der Ansprüche 1 bis 3, **dadurch gekennzeichnet, dass** die Innenseitenwand (216; 316) eine Innenschicht umfasst, die Silikon umfasst, die mit einer Außenschicht gekoppelt ist, die ein Material umfasst, das starrer als Silikon ist, und wobei optional das starre Material eines aus Polycarbonat, Polyamid und Copolyester ist.

5. Kombination von Zementformbaugruppen nach einem der Ansprüche 1 bis 4, **gekennzeichnet durch** eine Kopplung (343), die einen Fülldurchlass (345) in den Femurkomponentenformhohlraum (318) an einer Position bereitstellt, die quer zu einem Abschnitt der Gelenkfläche ist, der hauptsächlich mit einer Tibiaschale einer Tibiakomponente in Kontakt steht, und optional quer zu einem Abschnitt der Gelenkfläche ist, der hauptsächlich mit der Tibiaschale der Tibiakomponente in Kontakt steht.

6. Kombination von Zementformbaugruppen nach einem der Ansprüche 1 bis 5, **dadurch gekennzeichnet, dass** der längliche Formhohlraum (424, 425) für die den intramedulären Kanal füllende Komponente der Formbaugruppe für die den intramedulären Kanal füllende Komponente einen länglichen zylindrischen Formhohlraum (424) sowie einen einen reduzierten Durchmesser aufweisenden, sich axial erstreckenden Hohlraum (425) an einem Ende umfasst.

7. Kombination von Zementformbaugruppen nach Anspruch 6, **dadurch gekennzeichnet, dass** die Formbaugruppe (410) für die den intramedulären Kanal füllende Komponente eine gerundete Fläche (427) an einem Ende umfasst, das dem einen Ende entgegengesetzt ist, um ein Einsetzen einer geformten Schaftkomponente in einen länglichen Knochenhohlraum zu unterstützen.

8. Kombination von Zementformbaugruppen nach einem der Ansprüche 1 bis 7, **dadurch gekennzeichnet, dass** das flexible längliche Element der Formbaugruppe (410) für die den intramedulären Kanal füllende Komponente zumindest halbtransparent ist und Röntgenstrahlen durchlässt, die durch das flexible längliche Element betrachtet werden können.

9. Verfahren zum Herstellen eines temporären Knieimplantats, wobei das Verfahren die Schritte umfasst:
Erhalten einer Formbaugruppe (10; 110; 510) für eine Tibiakniekomponente, wobei die Formbaugruppe (10; 110; 510) für die Tibiakniekomponente ein erstes Formelement (12; 112; 512) und ein zweites Formelement (14; 114; 514) aufweist;
Einsetzen des zweiten Formelements (14; 114; 514) in das erste Formelement (12; 112; 512), um einen Formhohlraum (30; 136; 532) für die Tibiakniekomponente zu definieren, die einen eine Tibiaplattform bildenden Abschnitt und optional einen Schaft bildenden Abschnitt aufweist;
Vorschieben zumindest eines des ersten und zweiten Formelements (12, 14; 112, 114; 512, 514) zu dem anderen des ersten und zweiten Formelements (12, 14; 112, 114; 512, 514) durch aufeinanderfolgendes Ineingriffbringen zusammenwirkender Eingriffselemente (76; 575, 576) des ersten und zweiten Formelementes (12, 14; 112, 114; 512, 514), bis eine gewünschte Dicke des die Plattform bildenden Abschnittes erhalten ist;
Erhalten einer Formbaugruppe (210; 310) einer Femurkniekomponente, wobei die Formbaugruppe (210; 310) der Femurkniekomponente einen Femurkomponentenformhohlraum (218; 318) aufweist, der zwischen einer inneren Seitenwand (216; 316), die eine Form einer einem Knochen gegenüberliegenden Fläche definiert, sowie eine äußere Seitenwand (215, 314) gebildet ist, die eine Form einer Gelenkfläche zum Kontakt mit einer Tibiaschale einer Tibiakniekomponente definiert; und wobei das Verfahren durch den Schritt gekennzeichnet ist:
Erhalten einer separaten Formbaugruppe (410) einer einen intramedulären Kanal füllenden Komponente, wobei die Formbaugruppe (410) einer einen intramedulären Kanal füllenden Komponente einen länglichen Formhohlraum (424, 425) einer einen intramedulären Kanal füllenden Komponente aufweist, der durch ein flexibles längliches Element (422) definiert ist, das ermöglicht, dass der Formhohlraum (424, 425) so eingestellt wird, das er einem intramedulären Kanal eines individuellen Patienten entspricht.

10. Verfahren nach Anspruch 9, **gekennzeichnet durch**:
Einsetzen eines fließbaren, antibiotisch beladenen Knochenzementes in jeden des Formhohlraums der Tibiakniekomponente, des Formhohlraums der Femurkomponente und des Formhohlraums der den intramedulären Kanal füllenden Komponente durch einen Durchlass (38; 150; 345; 442; 538) in jeder der jeweiligen Formbaugruppe (10; 110; 510) der Tibiakniekomponente, der Formbaugruppe (210; 310) der Femurkomponente und der Formbaugruppe (410) der den intramedulären Kanal füllenden Komponente,
Entfernen der Tibiakniekomponente von der Formbaugruppe (10; 110; 510) der Tibiakniekomponente und der Femurkniekomponente von der Formbaugruppe (210; 310) der Femurkniekomponente, und der den intramedulären Kanal füllenden Komponente von der Formbaugruppe (410) der den intramedulären Kanal füllenden Komponente nach einem gewünschten Härten des fließbaren antibiotisch beladenen Knochenzementes in die relevante Formbaugruppe (10; 110; 210; 310; 410; 510).

11. Verfahren nach Anspruch 10, **dadurch gekennzeichnet, dass** ein Entfernen der Tibiakniekomponente umfasst, dass ein mit Gewinde versehenes Entfernungswerkzeug (523), das einen sich erstreckenden Vorsprung (524) umfasst, auf den Gewindeanschluss (538) der Formbaugruppe (510) der Tibiakniekomponente geschraubt wird, um gegen die Tibiakniekomponente zu drücken und diese von einem der Formelemente (512, 514) zu drängen.

12. Verfahren nach einem der Ansprüche 9 bis 11, **gekennzeichnet durch** ein Überlagern der Formbaugruppe (410) der den intramedulären Kanal füllenden Komponente mit einem Röntgen und Biegen des flexiblen länglichen Elements (422), um die tatsächliche Anatomie eines intramedulären Kanals eines spezifischen individuellen Patienten anzupassen, wie an dem Röntgen während des Aushärtens gezeigt ist.

13. Verfahren nach einem der Ansprüche 9 bis 12, **dadurch gekennzeichnet, dass** der längliche Formhohlraum (424, 425) der den intramedulären Kanal füllenden Komponente einen länglichen zylindrischen Formhohlraum (424) umfasst, und ein in der Abmessung reduzierter, sich axial erstreckender Hohlraum (425) einen Greifabschnitt an einem hinteren Ende bildet, wobei der Greifabschnitt gegriffen werden kann, um die den intramedulären Kanal füllende Komponente von einem intramedulären Kanal eines Patienten zu entfernen.

14. Verfahren nach einem der Ansprüche 9 bis 13, **gekennzeichnet durch** Platzieren von Knochenzement in einem teigigen Zustand zwischen einem hinteren Ende der den intramedulären Kanal füllenden Komponente, die von der Formbaugruppe (410) für die den intramedulären Kanal füllenden Komponente entfernt ist, und einer benachbarten Tibiakniekomponente, die von der Formbaugruppe (10; 110; 510) der Tibiakniekomponente entfernt ist, oder der Femurkniekomponente, die von der Formbaugruppe (210; 310) für die Femurkniekomponente entfernt ist.

## Revendications

1. Combinaison de trois assemblages de moules pour ciment séparés (10 ; 110 ; 210 ; 310 ; 410 ; 510), configurés chacun pour former un composant d'un implant de genou temporaire destiné à être utilisé pour distribuer des antibiotiques à un site infecté d'un genou, la combinaison comprenant :
un assemblage de moules pour composant de genou tibial (10 ; 110 ; 510), l'assemblage de moule pour composant de genou tibial ayant un second élément de moule (14 ; 114 ; 514) susceptible d'être reçu à l'intérieur d'un premier élément de moule (12 ; 112 ; 512) afin de définir une cavité de moule pour composant tibial (30 ; 136 ; 532) entre ceux-ci, la cavité de moule pour composant tibial (30 ; 136 ; 532) définissant une surface de plateau tibial et une surface d'appui tibial et en option une portion en forme de tige, et le second élément de moule (14 ; 114 ; 514) étant configuré pour être sélectivement et progressivement indexé par rapport au premier élément de moule (12 ; 112 ; 512) via des éléments d'engagement en coopération (76 ; 575, 576) jusqu'à obtenir une épaisseur désirée de la cavité de moule pour composant tibial (30) ;
un assemblage de moule pour composant de genou fémoral (210 ; 310), l'assemblage de moule pour composant de genou fémoral ayant une cavité de moule pour composant fémoral (218 ; 318) formée entre une paroi latérale intérieure (216 ; 316) définissant une forme d'une surface opposée à l'os et une paroi latérale extérieure (214 ; 314) définissant une forme d'une surface d'articulation qui est en contact avec un plateau tibial d'un composant tibial ;
la combinaison étant **caractérisée par**
un assemblage de moules pour composant de remplissage du canal intramédullaire (410), l'assemblage de moules pour composant de remplissage du canal intramédullaire (410) ayant une cavité de moule pour composant de remplissage du canal intramédullaire (424, 425) définie par un élément allongé flexible (422) permettant l'ajustement de la cavité de moule (424, 425) pour correspondre à un canal intramédullaire d'un patient individuel.

2. Combinaison d'assemblages de moules pour ciment selon la revendication 1, **caractérisée en ce que** la surface opposée à l'os tibial de l'assemblage de moules pour composant tibial (110 ; 510) comprend une surface texturée (154 ; 554).

3. Combinaison d'assemblages de moules pour ciment selon l'une quelconque des revendications 1 et 2, **caractérisée en ce que** l'un des éléments parmi le premier élément de moule (12 ; 112) et le second élément de moule (14 ; 114) de l'assemblage de moules pour composant tibial (10 ; 110) comprend des graduations (90 ; 132).

4. Combinaison d'assemblages de moules pour ciment selon l'une quelconque des revendications 1 à 3, **caractérisée en ce que** la paroi latérale intérieure (216 ; 316) comprend une couche intérieure comprenant du silicone couplé à une couche extérieure comprenant un matériau qui est plus rigide que le silicone et, en option, le matériau rigide est un matériau parmi un polycarbonate, un polyamide et un copolyester.

5. Combinaison d'assemblages de moules pour ciment selon l'une quelconque des revendications 1 à 4, **caractérisée par** un couplage (343) présentant un orifice de remplissage (345) vers l'intérieur de la cavité de moule pour composant fémoral (318) à une position qui est latérale par rapport à une portion de la surface d'articulation, qui vient principalement en contact avec un plateau tibial d'un composant tibial, et est en option latérale par rapport à la portion de la surface d'articulation qui vient principalement en contact avec le composant tibial du composant tibial.

6. Combinaison d'assemblages de moules pour ciment selon l'une quelconque des revendications 1 à 5, **caractérisée en ce que** la cavité de moule pour composant de remplissage du canal intramédullaire allongé (424, 425) de l'assemblage de moules pour composant de remplissage du canal intramédullaire comprend une cavité de moule allongée cylindrique (424) et une cavité de dimension réduite, s'étendant axialement (425) à une extrémité.

7. Combinaison d'assemblages de moules pour ciment selon la revendication 6, **caractérisée en ce que** l'assemblage de moules pour composant de remplissage du canal intramédullaire (410) comprend une surface arrondie (427) à une extrémité opposée à la première extrémité pour faciliter l'insertion du composant de tige moulé dans une cavité osseuse allongée.

8. Combinaison d'assemblages de moules pour ciment selon l'une quelconque des revendications 1 à 7, **caractérisée en ce que** l'élément allongé flexible de l'assemblage de moules pour composant de remplissage du canal intramédullaire (410) est au moins semi-transparent pour permettre de voir un rayon X à travers l'élément allongé flexible.

9. Procédé pour réaliser un implant de genou temporaire, le procédé comprenant les étapes consistant à :
obtenir un assemblage de moules pour composant de genou tibial (10 ; 110 ; 510), l'assemblage de moules pour composant de genou tibial (10 ; 110 ; 510) ayant un premier élément de moule (12 ; 112 ; 512) et un second élément de moule (14 ; 114 ; 514) ;
insérer le second élément de moule (14 ; 114 ; 514) dans le premier élément de moule (12 ; 112 ; 512) pour définir une cavité de moule pour composant de genou tibial (30 ; 136 ; 532) incluant une portion de formage pour plate-forme tibiale, et en option une portion de formage de tige ;
faire avancer l'un au moins du premier et du second élément de moule (12, 14 ; 112, 114 ; 512, 514) vers l'autre du premier et du second élément de moule (12, 14 ; 112, 114 ; 512, 514) en engageant successivement des éléments d'engagement coopérants (76 ; 575, 576) du premier et du second élément de moule (12, 14 ; 112, 114 ; 512, 514) jusqu'à obtenir une épaisseur désirée de la portion de formage de plate-forme ;
obtenir un assemblage de moules pour composant de genou fémoral (210 ; 310), l'assemblage de moules pour composant de genou fémoral (210 ; 310) ayant une cavité de moule pour composant fémoral (218 ; 318) formée entre une paroi latérale intérieure (216 ; 316) définissant une forme d'une surface opposée à l'os et une paroi latérale extérieure (214 ; 314) définissant une forme d'une surface d'articulation pour venir en contact avec un plateau tibial d'un composant de genou tibial ; et
le procédé étant **caractérisé par** l'étape consistant à :
obtenir un assemblage de moules séparé (410) pour composant de remplissage du canal intramédullaire, l'assemblage de moules pour composant de remplissage du canal intramédullaire (410) ayant une cavité de moule allongée pour composant de remplissage du canal intramédullaire (424, 425) définie par un élément allongé flexible (422) permettant un ajustement de la cavité de moule (424, 425) pour correspondre à un canal intramédullaire d'un patient individuel.

10. Procédé selon la revendication 9, **caractérisé par** les étapes consistant à:
introduire un ciment osseux liquide un chargé d'antibiotiques dans chacune des cavités parmi la cavité de moule pour composant de genou tibial, la cavité de moule pour composant fémoral, et la cavité de moule pour composant de remplissage du canal intramédullaire via un orifice (38 ; 150 ; 345 ; 442 ; 538) dans chacun des assemblages de moules respectifs parmi l'assemblage de moules pour composant de genou tibial (10 ; 110 ; 510), l'assemblage de moules pour composant fémoral (110, 310), et l'assemblage de moules (410) pour composant de remplissage du canal intramédullaire,
enlever le composant de genou tibial hors de l'assemblage de moules pour composant de genou tibial (10 ; 110 ; 510), et le composant de genou fémoral hors de l'assemblage de moules pour composant de genou fémoral (210, 310) et le composant de remplissage pour canal intramédullaire hors de l'assemblage de moules pour composant de remplissage de canal intramédullaire (410), et ultérieurement à un durcissement désiré du ciment osseux fluide chargé d'antibiotiques à l'intérieur de l'assemblage de moules concerné (10 ; 110 ; 210 ; 310 ; 410 ; 510).

11. Procédé selon la revendication 10, **caractérisé en ce que** l'enlèvement du composant de genou tibial comprend de visser un outil d'enlèvement fileté (523) comprenant une projection en extension (524) sur l'orifice taraudé (538) de l'assemblage de moule pour composant de genou tibial (510) afin de pousser contre et de séparer le composant de genou tibial depuis l'un des éléments de moule (512, 514).

12. Procédé selon l'une quelconque des revendications 9 et 11, **caractérisé en ce que** l'on superpose l'assemblage de moules pour composant de remplissage du canal intramédullaire (410) sur des rayons X et on cintre l'élément allongé flexible (422) pour s'accorder à l'anatomie réelle d'un canal intramédullaire d'un patient individuel spécifique, comme montré sur l'image en rayons X pendant le durcissement.

13. Procédé selon l'une quelconque des revendications 9 à 12, **caractérisé en ce que** la cavité de moule allongée pour composant de remplissage du canal intramédullaire (424, 425) comprend une cavité de moule cylindrique allongée (424), et une cavité (425) de dimension réduite et s'étendant axialement forme une portion de préhension au niveau d'une extrémité de queue où la portion de préhension peut être prise pour supprimer le composant de remplissage du canal intramédullaire depuis un canal intramédullaire d'un patient.

14. Procédé selon l'une quelconque des revendications 9 à 13, **caractérisé en ce que** l'on met le ciment osseux dans un état pâteux entre une extrémité de queue du composant de remplissage pour canal intramédullaire qui a été supprimé depuis le composant de remplissage du canal intramédullaire et l'assemblage de moules (410), et un composant adjacent parmi le composant de genou tibial supprimé de l'assemblage de moules pour composant de genou tibial (10 ; 110 ; 510) et le composant de genou fémoral supprimé hors de l'assemblage de moules pour composant de genou fémoral (210 ; 310).
